Europäisches Patentamt

European Patent Office

Office européen des brevets

(11) Publication number : **0 637 437 A1**

(12) **EUROPEAN PATENT APPLICATION**

(21) Application number : **94303110.4**

(22) Date of filing : **28.04.94**

(51) Int. Cl.$^6$ : **A61B 17/56**

(30) Priority : **14.05.93 US 62825**

(43) Date of publication of application :
**08.02.95 Bulletin 95/06**

(84) Designated Contracting States :
**AT BE CH DE DK ES FR GB GR IE IT LI LU MC NL PT SE**

(71) Applicant : **SMITH & NEPHEW RICHARDS, INC.**
**1450 Brooks Road**
**Memphis, Tennessee 38116 (US)**

(72) Inventor : **Small, Laura C.**
**239 Alexander Street**
**Memphis, Tennessee 38119 (US)**
Inventor : **Swain III, Robert E.**
**8624 Riverchase Drive**
**Germantown, Tennessee 38139 (US)**

(74) Representative : **Gilholm, Stephen Philip et al**
**Corporate Patents Department**
**Smith & Nephew Group Research Centre**
**York Science Park**
**Heslington York YO1 5DF (GB)**

(54) **Composite spinal apparatus.**

(57)  A composite surgical spinal fixation system including a bone bolt (11) having end portions (12) that are adapted to be surgically implanted into a patient's spine at first and second spaced apart positions and a central portion of the bone bolt defining a load transfer surface, the bone bolt having a second upper threaded section (14) for threadably receiving a nut (16) at a shaped, countersunk interface (42). As an alternate embodiment, a bone screw provides an upper enlarged head (46) with a lower coarsely threaded shank (44). A composite plate (17) having upper and lower surfaces and outer opposed edges is provided with an elongated slot (20) for accommodating a selected bone bolt or bone screw. The plate edges carry fine adjustments (27,28) extending between the upper and lower surfaces of the plate and a load transfer washer (35) interfaces the plate and the selected bone bolt or bone bolt (or screw) to the plate. The composite plate adopts a curved configuration via asymmetric ply lamination or by melt-forming. The curvature of the plate is dictated by the natural lordotic curvature of the patient's spine. The plate is comprised of either a plurality of laminal arranged in a specific ply sequence or a plurality of fibers or tows arranged through winding and/or placement. Electrochemical compatibility with the composite plate is ensured by careful selection of the materials comprising the attachment means and the washer mechanism.

FIG.1.

The present invention relates to surgical systems and more particularly relates to an improved composite spinal plate apparatus in the form of a strong and stable construct for maximum fusion augmentation with improved versatility and ease of use, and wherein a washer forms a load transfer interface with the composite plate and one or more adjacent non-metallic (eg. ceramic or composite), or metallic fixation screws. A countersunk interface provides some micro-motion, better stress distribution and thus enhanced fatigue life.

There are a number of surgical procedures which require a fixation of portions of the spine with respect to one another. Frequently, bone screws are employed in the fixation of the spine. The implantation of bone screws is a surgical procedure which involves the formation of one or more surgical openings in adjacent portions of the spine, with threaded bone screws being implanted into these surgical openings. Connective structures such as rods or plates extend between the various spine members by connecting the adjacent bone screws.

An early spinal fixation system can be seen in the Lumb et al patent 3,648,691 entitled "Method of Applying Vertebral Appliance". In the Lumb patent, a method of applying a vertebral appliance for use in bridging one or more diseased or damaged vertebrae uses a pair of elongated flexible multiple aperture plates having fasteners which are used to clamp the plate to opposite sides of the spinous processes being spanned. Each strap or plate is of a length adapted to span at least two spinous processes and project beyond each end so that the fasteners can be passed both behind and in front thereof as well as through the interspinous gap there between. The apertures are located considerably closer together than adjacent processes and they are fastened to the latter in position such that at least one opening registers with each one to receive a growth or soft bony tissue that eventually extrudes therein.

A polymer composite that was evaluated for use an orthopaedic, variable screw placement (VSP) spinal plate is disclosed in a 1989 article entitled "Accelerated Testing of A Composite Spine Plate". It was felt by the authors that fretting fatigue or cyclic loading that causes micromotion at interfaces in the spine plate could cause significant wear of the composite plate. Therefore, a test method was developed to assess a continuous carbon fibre polysulphone composite plate in *in vitro* fretting fatigue. A relevant failure criterion was established and employed to determine the effect of torque on the fatigue life of the plate. Two failure mechanisms were observed and the plate displacement behaviour was indicative of these mechanisms. The authors felt that an optimum implant torque was defined. The authors felt that the results demonstrated the importance of tailoring the material to the implant application. The article discusses several material improvements as having being implemented.

The Edwards patent 4,369,769 shows a spinal fixation system using elongated rods used to bridge across various portions of the spine. In the Edwards '769 patent a spinal fixation device is provided in which sleeves or spacers are placed over and around spinal rods in order to obtain a better reduction of spinal fractures or spinal deformities. These sleeves can be made in various thicknesses so that the surgeon can obtain optimum fixation in each case. The sleeves are made of any biologically compatible material.

Use of bone screws and connecting rods is also seen in the Ulrich et al patent 4,433,677 entitled "Implantable Splint for Correction Lumbosacral Spondylodesis". In the Ulrich patent a spinal distraction splint has two like anchor screws extending along respective longitudinal screw axes and adapted to be anchored in the pelvis with the axes crossing. Each of the screws has a head formed with a transverse open recess centered on respective transverse axis and with an angular array of teeth centered on and angularly spaced about the respective transverse axis.

Another patent that shows screws as part of a spinal stabilizer is the Stephens et al patent 4,604,995. In the Stephens patent a surgical implant is used for imparting stability to the thoraco-lumbar spine by fixation of the implant to the spine with segmental spinal instrumentation. The implant comprises a unitary rod having a generally rectangular configuration formed by a pair of spaced apart branches, mirror image duplicated of one another and equally spaced apart along their length.

The Steffee patent 4,611,581 entitled "Apparatus for Straightening Spinal Columns" provides an apparatus to reduce the extent of displacement between adjacent vertebra in a person's spinal column and to subsequently maintain the vertebra in a reduced displacement relationship. When the apparatus is to be installed, holes are formed in the displaced vertebra and in vertebra on opposite sides of the displaced vertebra. Force transmitting members are mounted in the holes in the vertebra. A spinal plate is then positioned on the spinal column with the force transmitting members extending outwardly through the slots in the spinal plate. Nuts are tightened on the force transmitting members connected with vertebra on opposite sides of the displaced vertebra to anchor the spinal plate in place. A nut on the force transmitting member connected with the displaced vertebra is then tightened to pull the displaced vertebra to a desired position. In one embodiment, the force transmitting member has a relatively large diameter helix which engages a side wall of the hole in the displaced vertebra. In another embodiment, an insert is positioned in a hole in the displaced vertebra and expanded by the force transmitting member to securely grip the vertebra.

A device which uses clamps as opposed to bone screws is the Asher patent 4,773,402 entitled "Dorsal Transacral Surgical Implant" wherein a pair of spine engageable rods, contoured to the desired spinal column configuration are provided with a yoke and foot element being attached to the pair of rods during use.

The Sherman patent 4,887,596 shows a pedicle screw for use in internal fixation of the spine comprising a shaft threaded at one end for insertion into a bone and at the other end having a yoke for receiving a rod, the yoke having a cusp adapted to bear against the rod and clamps for holding the rod against the cusp while permitting adjustment of the angle between the rod and the yoke.

Transpedicular plates have become a popular choice among leading surgeons when indications call for fusion in the lumbosacral region of the spine. The pedicle screw-plate construct lends the surgeon a great deal of strength and stability which translates into a high incidence of successful operative treatment.

The transpedicular spinal plating concept is not without its shortcomings. The proximate position of the spinal cord and nerve roots to the pedicle leaves the surgeon with no room for screw misplacement. The very benefit of construct stability, ie. the plate's ability to restrict segmental motion, can betray the efficacy of the construct by inducing stress-related osteopenia, which, in turn, may lead to hardware failure.

This particular problem could be remedied by replacing inordinately stiff constructs with more compliant systems. A delicate balance exists, however, between construct stiffness and the rate of bony fusion. The fixation device should be "stiff enough to limit displacement and strain, strong enough so that it will not break, and supple enough so that it will not cause breakdown at the bone-implant interface".

All presently available lumbosacral spinal plating systems on the market are fabricated of a metallic material. This fact does not necessarily dictate the level of stiffness achieved by a spinal construct. It does, however, place practical limits on the amount of device compliance designed into the plating system, since metals generally have high stiffness (low compliance) compared to most other materials. In other words, barring designs that exploit geometrical deformation, there exists an exceptionally low maximum compliance level that metallic structures are physically unable to surpass. Having a material system in which one could dictate the level of stiffness inherent in the material would be a significant attribute in this regard. The biological effects of wear debris and ion release arising in metallic components has come under scrutiny. In addition, in this age of sophisticated imaging systems, it is quite common for a surgeon to require a combination of x-ray, computer tomographic, and magnetic resonance images to complete the diagnosis and suggest the most time-effective course for recovery. Metallic plating systems inhibit such imaging. They remain opaque to x-ray, and distort CT and MR images.

The deficiencies of current metallic spinal plating systems identify a number of the problems that need to be solved. First, a material system should enable the designer to dictate the construct stiffness, instead of allowing the metallic system to dictate the stiffness level. Secondly, a material system should not comprise a surgeon's ability to employ all imaging techniques. Finally, a material system should match, if not improve upon, the biocompatibility of current metallic devices.

The present invention solves these prior art problems and shortcomings by providing a spinal plate apparatus of composite material, such as carbon fiber/PEEK composite. The apparatus of the present invention is compliant, biocompatible, radiolucent, and minimizes distorted artifacts from CT and MR imaging.

The present invention provides an improved bone fixation apparatus that includes a bone attachment means that has an end portion that is adapted to be surgically implanted into a patient's bone tissue. The attachment means can be a bone bolt (see Figures 1 and 3) or a bone screw (see Figure 9). A fiber reinforced composite plate member includes upper and lower surfaces and parallel outer opposed edges, with an elongated slot that is sized and shaped to allow the placement of the bone attachment means thereunto, and surrounded by a peripheral portion having the parallel outer opposed edges.

Thus according to the invention we provide a bone fixation apparatus, comprising:

a) a fastener having an end portion adapted to be surgically implanted into a patient's bone tissue;

b) a fiber reinforced composite plate member having upper and lower surfaces and outer opposed edges with an elongated slot sized and shaped to allow placement of the fastener thereunto, and surrounded by a peripheral portion having said outer proposed edges; and

c) the plate member carrying adjustment means for affixing the position of the fastener with respect to the plate.

We further provide a composite bone plate system comprising:

a) at least one bone attachment means having an end portion adapted to be surgically implanted into a patient's bone tissues;

b) a portion of the bone attachment means having a longitudinally extending, shaped load transfer surface;

c) a composite bone plate member having upper and lower surfaces and outer opposed edges with an elongated slot surrounded by a peripheral portion having said parallel outer opposed edges, the slot being sized to receive the bone attachment means;

EP 0 637 437 A1

d) a washer having an underside portion with projections extending therefrom; and

e) the plate member carrying adjustment means and each of the projections sized and shaped to fit selected of said adjustment means.

The plate member includes fine adjustment openings in the preferred embodiment that extend between the upper and lower surfaces for affixing the position of the bone attachment means with respect to the plate.

In a yet further embodiment we provide a composite bone plate system, comprising:

a) at least one bone attachment means having an end portion adapted to be surgically implanted into a patient's bone tissues;

b) a portion of the bone attachment means having a longitudinally extending, shaped load transfer surface;

c) a composite bone plate member having upper and lower surfaces and outer opposed edges with an elongated slot surrounded by a peripheral portion having said parallel outer opposed edges, the slot being sized to receive the bone attachment means;

d) a washer having an underside portion with projections extending therefrom; and

e) the plate having a plurality of adjustment position openings and each of the projections sized and shaped to fit selected of the adjustment openings.

The adjustment means includes a load transfer washer that interfaces the plate member and the bone attachment means. The washer has means for distributing load from the attachment means (ie. bone bolt or bone screw) to the plate member and portions of the washer engage an opening in the plate.

The plate is preferably a fiber reinforced structure having fibers that are oriented at zero degree (0°), forty five degrees (45°), ninety degrees (90°), and one hundred thirty five degrees (135°).

The composite plate has a lower surface that can curve in order to conform to the natural lordotic curvature of the lumbosacral spine.

The composite plate is preferably fiber reinforced and comprised of a molded composite plate structure having at least one of said upper and lower surfaces that can be curved.

The reinforced composite plate member can be comprised of a plurality of laminates arranged in an asymmetric ply sequence, defining a curvature for one or both of the upper and lower surfaces that conform to the natural lordotic curvature of the lumbosacral spine. The reinforced composite plate member can be comprised of a plurality of fibers or tows arranged through winding and/or placement such that a curved upper and/or lower surface is established.

The load transfer washer can include a washer that has a first flange that extends across the slot between the edge portions thereof. The apparatus can be used with bone screws or with bone bolts, and may be metallic or non-metallic.

In the event that the reinforcing phase of the composite plate is an electron conductor, it is imperative that the washer and bone attachment means be comprised of materials that ensure electrochemical compatibility with the composite plate once implanted in the electrolytic physiological environment. Thus, if carbon (or graphite) fibers reinforce the composite plate, in order to preclude the formation of a galvanic corrosion cell, the apposing devices should be non-metallic (electron insulators) or composed of materials with similar electrode potentials.

In the preferred embodiment, the fine adjustment can be provided in the form of indentations that are positioned along the edge portions of the composite plate.

It is thus an object of the present invention to provide a composite plate fixation system that offers a strong and stable construct for maximum fusion augmentation and yet is versatile enough for any patient and is easy to use.

The composite spinal plating system of the present invention provides a material that has improved compliance, improved biocompatibility, and which does not interfere with the surgeon's ability to employ all imaging techniques.

The apparatus provides a composite plate member having upper and lower surfaces and generally parallel outer opposed edges with an elongated slot extending along the longitudinal center line of the composite plate member. The slot is sized and shaped to allow placement of a bone attachment means thereunto wherein the bone attachment means is surrounded by a peripheral portion which communicates with the parallel outer opposed edges.

A bone bolt, preferably non-metallic (eg. ceramic or composite) provides a first end portion that is adapted to be surgically implanted into a patient's bone tissue. A second end portion of the bone bolt forms an attachment to a load transfer washer. The load transfer washer interfaces the plate member and the bone bolt and distributes load from the bone bolt to the plate member. A fine adjustment is provided for positioning the load transfer washer upon the plate. In another embodiment, the washer has an underside that includes one or more projections. The plate provides a series of spaced-apart openings that are receptive of the projections of the washer. This combination of the projections on the washer and the openings in the plate form a connection as

4

well as a means for adjustment between the washer and plate. The washer clamps to the plate with a force sufficient to enable the load to be transferred to the plate. The combination of the projections and the openings in the plate define a supplemental mechanism to transfer loads between the bone attachment means and the plate. The other mechanism for transferring load is the transfer of shear stresses arising from friction.

The spinal plate of the present invention can endure compressive and tensile loads, along with considerable bending and torsion. The use of composite materials allows flexibility to respond to specific loads encountered. In the preferred configuration, a plate structure is provided possessing fibers that are oriented at zero degree (0°), forty five degrees (45°), ninety degrees (90°), and one hundred thirty five degrees (135°) to the longitudinal axis of the plate. This orientation provides strength and stiffness to the plate when loaded in a variety of modes.

The plate apparatus of the present invention can provide a plate that is adapted to be curved in order to conform to the natural lordotic curvature of the lumbosacral spine. This can be achieved by one of the following two methods. First, a curved configuration could be imparted to the plate via a curved mold. When the plate is being consolidated under conditions of high temperature and pressure, a curved mold could be used to force the part into its final shape. The curvature can be formed by laminating with an asymmetric ply sequence. For instance, if the upper half of the plate is more susceptible to contraction during processing cool down the bottom half of the plate, then the plate will "warp" around the more susceptible half to a permanent curved shape.

One embodiment of the apparatus of the present invention provides location of the supplemental means by which load is transferred to the plate. Instead of placing the engaging holes centrally along the two ligaments of the plate, the holes are moved such that the centre of the holes coincide with the edge of the plate. To correspond to this change, the washer now incorporates a mating arcuate, semi-circular region.

A countersunk interface can be located between a washer and nut. This improved construction provides some micromotion, better stress distribution, and thus enhanced fatigue life.

Another object of the present invention is to provide an improved composite spinal fixation apparatus having improved fit through the use of a fine adjustment between adjacent bone attachment means. This high resolution allows each bone screw and/or bolt to be placed anatomically with little manipulation to make the plate fit the bone screws of the system.

We also provide a bone fixation system, comprising:

a) a bone attachment means having an end portion adapted to be surgically implanted into a patient's bone tissue and a section of the bone attachment means having a load transfer;

b) a composite plate member of fiber reinforced material having upper and lower surfaces and parallel outer opposed edges with an elongated slot surrounded by a peripheral portion having said parallel outer opposed edges;

c) a load transfer washer interfacing the plate member and the bone attachment means for distributing load from the bone via the attachment means to the plate member; and

d) an opening in the washer for receiving the bone attachment means therethrough.

A plurality of fiber reinforcements can be stacked asymmetrically to provide a curvature so that the plate conforms to the natural curvature of the spine.

The plate portion of the preferred embodiment and the alternative embodiment are each of a composite material which is radiolucent. Accordingly, radiolucent washers and screws can be used with the plate portion.

The washer preferably provides a downwardly extending projecting peg portion which engages grooved edges of the edge portion of the plate. In the alternate embodiment, the peg portion engages holes that are spaced between a central slot of the plate and the edge portion of the plate.

Therefore, the present invention provides an improved bone fixation apparatus of a composite radiolucent material as well as radiolucent washers and bone attachment means that engage those plates.

We also provide a load transfer washer adapted to interface with a plate member and a bone attachment means said washer being provided with an underside portion with projections extending therefrom adapted to correspond with openings in the limbs of said plate member.

For a further understanding of the nature and objects of the present invention, reference should be made to the following detailed description taken in conjunction with the accompanying drawings, in which like parts are given like reference numerals, and wherein:

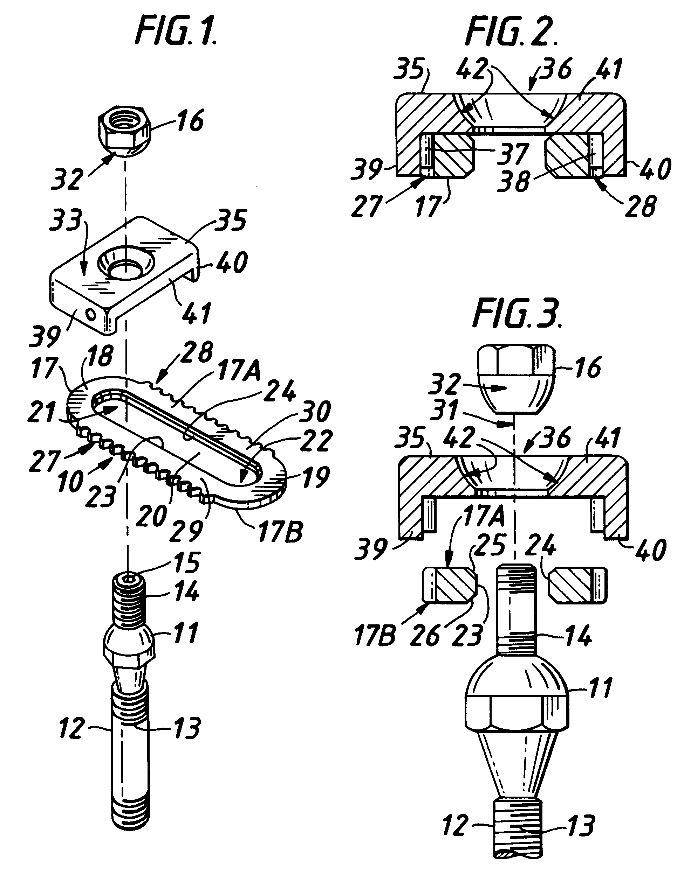

Figure 1 is an exploded perspective view of the preferred embodiment of the apparatus of the present invention shown in use with a bone bolt;

Figure 2 is a partial sectional, elevational view of the preferred embodiment of the apparatus of the present invention;

Figure 3 is a sectional, elevational view of the preferred embodiment of the apparatus of the present invention;

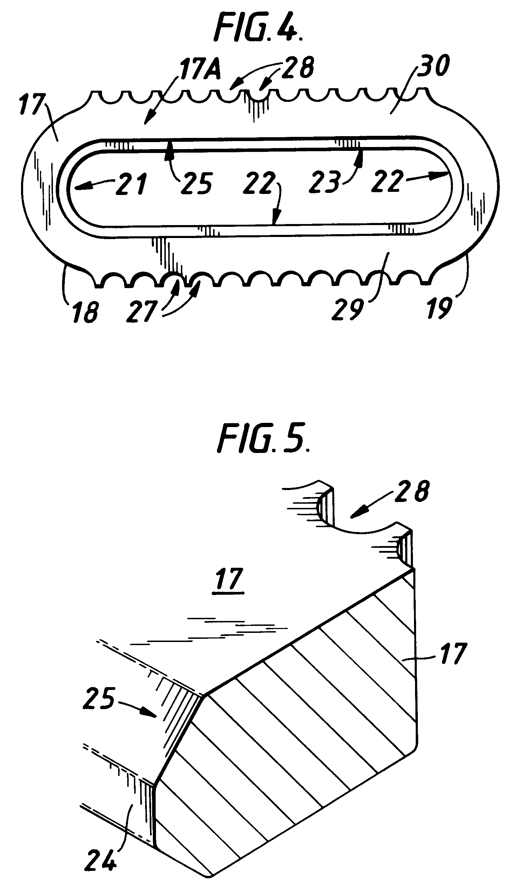

Figure 4 is a top fragmentary view of the preferred embodiment of the apparatus of the present invention;

Figure 5 is a fragmentary perspective view of the preferred embodiment of the apparatus of the present invention illustrating the plate portion thereof;

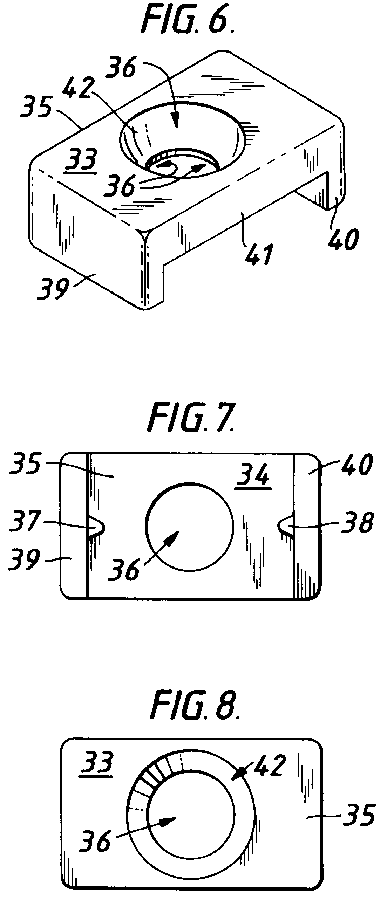

Figure 6 is a partial perspective view of the preferred embodiment of the apparatus of the present invention illustrating the washer portion thereof;

Figure 7 is a partial bottom view of the preferred embodiment of the apparatus of the present invention illustrating the washer portion thereof;

Figure 8 is a partial top view of the preferred embodiment of the apparatus of the present invention illustrating the washer portion thereof;

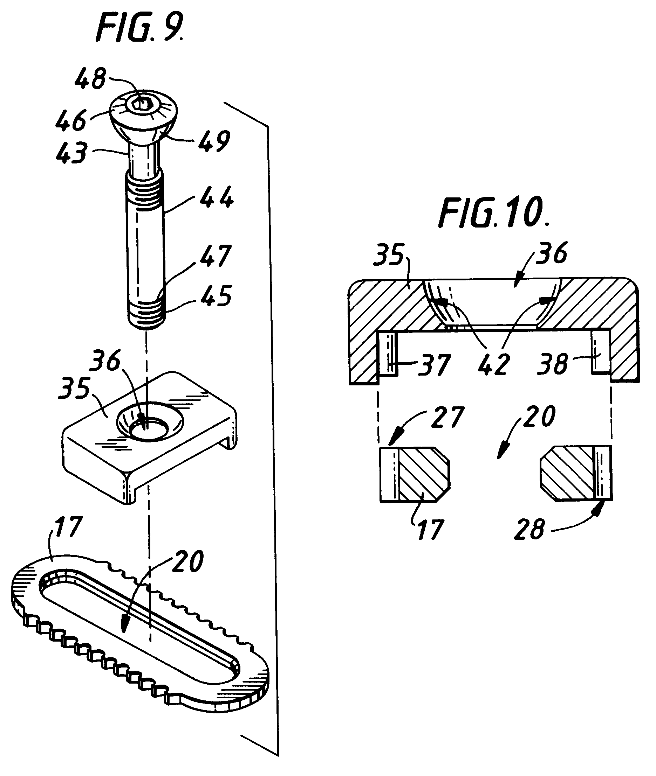

Figure 9 is a perspective exploded view of the preferred embodiment of the apparatus of the present invention shown in use with a bone screw;

Figure 10 is a partial sectional elevational view of the bone plate and washer portion of the preferred embodiment of the apparatus of the present invention as used in Figure 9;

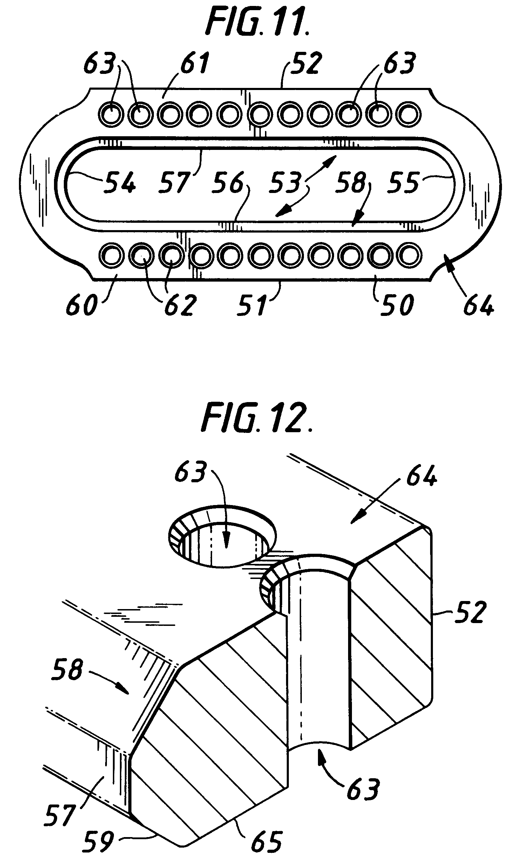

Figure 11 is a top view of the plate portion of an alternate embodiment of the apparatus of the present invention;

Figure 12 is a fragmentary perspective view of the plate portion of an alternate embodiment of the apparatus of the present invention;

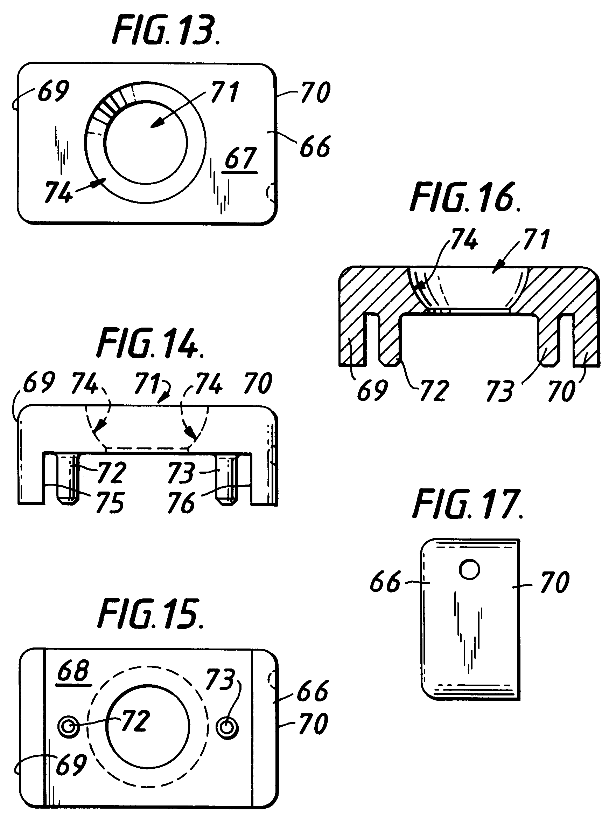

Figure 13 is a top view of the washer portion of the alternate embodiment of the apparatus of the present invention;

Figure 14 is a front view of the washer portion of the alternate embodiment of the apparatus of the present invention;

Figure 15 is a bottom view of the washer portion of the alternate embodiment of the apparatus of the present invention;

Figure 16 is a sectional elevational view of the washer portion of the alternate embodiment of the apparatus of the present invention; and

Figure 17 is a side view of the washer of Figures 13-16.

Figures 1 - 8 illustrate the preferred embodiment of the apparatus of the present invention designated generally by the numeral 10. In Figure 1 there can be seen bone bolt 11 having a shank portion 12 with a coarse thread 13 thereon. At the upper end portion of bone bolt 11, finely threaded head portion 14 is provided, with a tool receptive socket 15. A hexagonal tool socket 15 is preferably provided at the upper end portion of bone screw 11 for receiving an allen wrench or like tool for applying torque for installation of the screw threads 13 into a surgical opening of the patient's bone tissue. A nut 16 having hemispherically shaped underside is provided for forming a load transfer interface with washer 35. The nut 16 has fine threads that engage corresponding threads on the head portion 14 of bone bolt 11.

Plate 17 includes an upper surface 17A and a lower surface 17B. The plate 17 is elongated and provides curved end portions 18, 19. A longitudinally extending slot 20 includes semicircular end portions 21, 22. Slot 20 is surrounded by parallel side walls 23, 24. The slot 20 can include an upper beveled surface portion 25 and a lower beveled surface portion 26 (Figure 3) which communicate respectively with the slot at the top 17A and bottom 17B of plate 17. The beveled surface portions 25, 26 can interface with similarly shaped surfaces of a bone bolt 11.

A pair of limb portions 29, 30 are provided to plate 17 on the sides of slot 20. The limbs 29, 30 of plate 17 each include a plurality of spaced apart semicircular notches 27, 28 respectively. Notches 27, 28 each define an adjustment position. The notches are spaced apart, for example, by the distance of just a few millimeters.

Washer 35 includes side walls 39, 40 which are spaced apart and which connect to washer transverse member 41. An opening 36 in washer 35 extends between upper surface 33 and lower surface 34. Opening 36 is defined by hemispherical concave surface 42 that is similarly shaped to the hemispherical convex surface 32 of nut 16 to form an interface therewith during use. Projections 37, 38 are correspondingly sized and shaped to register with the notches 27, 28 in plate 17 when the central axis 31 of opening 36 aligns with the central longitudinal axis of slot 20.

In Figures 9 - 10, a bone screw 43 is shown having a shank portion 44, a lower distal end 45, and a head portion shaped section 46. A coarse thread 47 extends along the length of shank 44 below the frustoconical section 46. The coarse thread 47 provides bone engaging threads for surgically inserting the shank 44 portion of bone screw 43 into the patient's bone tissue. A hexagonally shaped socket 48 provides a means for imparting torque to the bone screw 43 using a tool. Hemispherically shaped surface 49 is positioned to engage similarly shaped surface 42 of washer 35.

In Figures 11 - 17, alternate embodiments of the bone fixation plate (Figures 11 - 12) and washer (Figures 13 - 17) are shown. In Figures 11 - 12, composite plate 50 includes a plate that has generally flat, parallel opposed outer walls 51, 52. As with the preferred embodiment, plate 50 provides a centrally longitudinally extending slot 53. The slot 53 includes generally semicircular end portions 54, 55. The slot is defined by a pair of generally parallel opposing sides 56, 57 and by the semicircular end portions 54, 55 as shown in Figure 11. Plate 50 provides an upper beveled surface 58 and a lower beveled surface 59, each communicating with slot 53. The plate 50 includes limbs 60, 61. Each of the limbs 60, 61 carries a plurality of spaced apart openings 62, 63 respectively. Each of the openings 62, 63 extends between a plate upper surface 64 and a plate lower surface 65. Plate 50 is used with washer 66. Washer 66 has an upper surface 67, a lower surface 68 and side walls 69, 70. Washer 66 has a central opening 71 that extends between upper surface 67 and lower surface 68. A pair of cylindricallly shaped projections 72, 73 extend below the bottom surface 68 of washer 66 as shown in Figures 14 - 16. Projections 72, 73 are sized and shaped and spaced apart so that when washer 66 engages plate 50, the projections 72, 73 register with a selected opening 62 and a selected opening 63. During use, washer 66 is placed upon plate 50. The projections 72, 73 register in a selected opening 62 and a selected opening 63 respectively. Flat surfaces 75, 76 of washer 66 align with the surfaces 51, 52. Opening 71 of washer 66 provides a hemispherically shaped surface of a nut or a bone screw as with respect to the preferred embodiment of Figures 1 - 8.

The spinal plate of the present invention can endure compressive and tensile loads, along with considerable bending and torsion. The use of composite materials allows flexibility to respond to specific loads encountered. In the preferred configuration, a plate is provided possessing fibers that are oriented at zero degree (0°), forty five degrees (45°), ninety degrees (90°), and one hundred thirty five degrees (135°) to the longitudinal axis of the plate. This orientation provides strength and stiffness to the plate when loaded into a variety of modes.

The plate portion of the preferred embodiment and the alternate embodiment are each of a composite material which is radiolucent. Accordingly, radiolucent washers and screws can be used with the plate portion.

The washer preferably provides a downwardly extending peg portion which engages correspondingly shaped grooved edges of the plate.

In the alternate embodiment, the peg portion engages holes that are spaced between a central slot of the plate and the edge portion of the plate.

Table 1 below lists a summary of the parts including the part number and corresponding description as used herein and in the drawings.

## Table 1
## PARTS LIST

| Part No. | Description |
| --- | --- |
| 10 | bone fixation apparatus |
| 11 | bone bolt |
| 12 | shank |

| 13 | coarse thread |
|----|----|
| 14 | head |
| 15 | nut |
| 16 | convex surface |
| 17 | plate |
| 17A | upper surface |
| 17B | lower surface |
| 18 | curved end of plate |
| 19 | curved end of plate |
| 20 | slot |
| 21 | semicircular end wall |
| 22 | semicircular end wall |
| 23 | parallel side walls |
| 24 | parallel side walls |
| 25 | bevelled edges (upper) |
| 26 | bevelled edges (lower) |
| 27 | semicircular notch |
| 28 | semicircular notch |
| 29 | limb |
| 30 | limb |
| 31 | axis |
| 32 | hemispherical convex surface |
| 33 | top surface |
| 34 | bottom surface |
| 35 | washer |
| 36 | opening |
| 37 | projection |
| 38 | projection |
| 39 | sidewall of washer |
| 40 | sidewall of washer |
| 41 | transverse member |
| 42 | hemispherical concave member |
| 43 | bone screw |
| 44 | shank |
| 45 | distal end |
| 46 | head |
| 47 | coarse thread |
| 48 | hexagonal socket |

| 49 | hemispherical section |
| 50 | bone fixation plate |
| 51 | side wall |
| 52 | side wall |
| 53 | slot |
| 54 | semicircular end portion |
| 55 | semicircular end portion |
| 56 | side wall |
| 57 | side wall |
| 58 | beveled surface |
| 59 | beveled surface |
| 60 | limb |
| 61 | limb |
| 62 | opening |
| 63 | opening |
| 64 | upper surface |
| 65 | lower surface |
| 66 | washer |
| 67 | upper surface |
| 68 | lower surface |
| 69 | side wall |
| 70 | side wall |
| 71 | opening |
| 72 | cylindrical projection |
| 73 | cylindrical projection |
| 74 | hemispherical surface |
| 75 | flat surface |
| 76 | flat surface |

Because many varying and different embodiment may be made within the scope of the inventive concept herein taught, and because many modifications may be made in the embodiments herein detailed in accordance with the descriptive requirement of the law, it is to be understood that the details herein are to be interpreted as illustrative and not in a limiting sense.

**Claims**

1. A bone fixation apparatus, comprising:
   a) a fastener having an end portion adapted to be surgically implanted into a patient's bone tissue;
   b) a fiber reinforced composite plate member having upper and lower surfaces and outer opposed edges with an elongated slot sized and shaped to allow placement of the fastener thereunto, and surrounded by a peripheral portion having said outer opposed edges; and
   c) the plate member carrying adjustment means for affixing the position of the fastener with respect to the plate.

2. An apparatus according to claim 1 wherein the composite plate has a lower surface that can be curved in order to conform to the natural lordotic curvature of the lumbosacral spine.

3. An apparatus according to claim 2 wherein the fiber reinforced composite plate is comprised of a molded composite structure having one of said upper and lower surfaces that can be curved.

4. An apparatus according to claim 2 wherein the fiber reinforced composite plate member is comprised of a plurality of laminae arranged in an asymmetric ply sequence, defining a curvature for one or both of the upper and lower surfaces that conform to the natural lordotic curvature of the lumbosacral spine.

5. An apparatus according to claim 2 wherein the fiber reinforced composite plate member comprises a plurality of fibers or tows arranged through winding and or placement so that a curved upper or lower surface is formed.

6. An apparatus according to claim 1 wherein the load transfer washer includes a washer having a first flange that extends across the slot between the edges.

7. An apparatus according to claim 1 wherein there are at least a pair of bone fasteners and the slot is sized to accommodate the bone fasteners when spaced apart and surgically implanted.

8. An apparatus according to claim 1 wherein the fine adjustment means includes indentations spaced along the edge portions of the plate.

9. An apparatus according to claim 8 wherein the fine adjustment means includes regularly spaced teeth spaced along each of the edges with indentations therebetween, and corresponding projections on the washer for engaging the indentations.

10. An apparatus according to claim 1 wherein the slot has a central axis and the washer has an opening therein with a center that registers with the central axis of the slot during use.

11. An apparatus according to claim 1 wherein the washer includes a recess that registers with the plate so that the washer slides upon the plate at the recess.

12. An apparatus according to claim 1 wherein the washer has three intersecting flange portions including a center flange and two side flanges that are spaced apart and positioned to bear against the edges of the plate member.

13. An apparatus according to claim 1 wherein the plate member comprises a pair of limbs said limbs defining the elongated slot and fine adjustment means comprises a plurality of openings in said limbs.

14. A composite bone plate system comprising:
    a) at least one bone attachment means having an end portion adapted to be surgically implanted into a patient's bone tissues;
    b) a portion of the bone attachment means having a longitudinally extending, shaped load transfer surface;
    c) a composite bone plate member having upper and lower surfaces and outer opposed edges with an elongated slot surrounded by a peripheral portion having said parallel outer opposed edges, the slot being sized to receive the bone attachment means;
    d) a washer having an underside portion with projections extending therefrom; and
    e) the plate member carrying adjustment means and each of the projections sized and shaped to fit selected of said adjustment means.

15. A composite bone plate system, comprising:
    a) at least one bone attachment means having an end portion adapted to be surgically implanted into a patient's bone tissues;
    b) a portion of the bone attachment means having a longitudinally extending, shaped load transfer surface;
    c) a composite bone plate member having upper and lower surfaces and outer opposed edges with an elongated slot surrounded by a peripheral portion having said parallel outer opposed edges, the slot being sized to receive the bone attachment means;

d) a washer having an underside portion with projections extending therefrom; and

e) the plate having a plurality of adjustment position openings, and each of the projections sized and shaped to fit selected of the adjustment openings.

16. A system according to claim 15 wherein the composite plate has a lower surface that is curved in order to conform to the natural lordotic curvature of the lumbosacral spine.

17. A system according to claim 16 wherein the fiber reinforced composite plate is comprised of a molded composite plate having one of said upper and lower surfaces that is curved.

18. A system according to claim 16 wherein the fiber reinforced composite plate member is comprised of a plurality of laminae arranged in an asymmetric ply sequence, defining a curvature for one or both of the upper and lower surfaces that conform to the natural lordotic curvature of the lumbosacral spine.

19. A system according to claim 16 wherein the fiber reinforced composite plate member comprises a plurality of fibers or tows arranged through winding and or placement so that a curved upper or lower surface is formed.

20. A system according to claim 15 wherein the load transfer washer has a first flange that extends across the slot between the edges and a pair of intersecting flanges extending at angles thereto.

21. A system according to claim 15 wherein the slot is sized to accommodate at least a pair of spaced apart and surgically implanted bone attachment means.

22. A system according to claim 15 wherein the adjustment means includes a plurality of semicircular openings spaced along the edges.

23. A system according to claim 15 wherein the washer has three intersecting flange portions including a center flange and two side flanges that are spaced apart and positioned against the edges of the plate member.

24. A system according to claim 15 wherein the washer has an opening with a hemispherical portion that receives a corresponding hemispherical portion of the bone attachment means.

25. A system according to claim 15 wherein the slot has a lower beveled surface and the bone bolt has a corresponding surface that abuts the beveled surface for load transfer.

26. A bone fixation system, comprising:

a) a bone attachment means having an end portion adapted to be surgically implanted into a patient's bone tissue and a section of the bone attachment means having a load transfer surface;

b) a composite plate member of fiber reinforced material having upper and lower surfaces and parallel outer opposed edges with an elongated slot surrounded by a peripheral portion having said parallel outer opposed edges;

c) a load transfer washer interfacing the plate member and the bone attachment means for distributing load from the bone via the attachment means to the plate member; and

d) an opening in the washer for receiving the bone attachment means therethrough.

27. A system according to claim 26 wherein the composite plate has a lower surface that is curved in order to conform to the natural lordotic curvature of the lumbosacral spine.

28. A system according to claim 27 wherein the fiber reinforced composite plate is comprised of a molded composite plate having one of said upper and lower surfaces that is curved.

29. A system according to claim 27 wherein the fiber reinforced composite plate member is comprised of a plurality of laminae arranged in an asymmetric ply sequence, defining a curvature for one or both of the upper and lower surfaces that conform to the natural lordotic curvature of the lumbosacral spine.

30. A system according to claim 27 wherein the fiber reinforced composite plate member comprises a plurality of fibers or tows arranged through winding and or placement so that a curved upper or lower surface is formed.

**31.** An apparatus according to claim 1 wherein the bone attachment means is metallic.

**32.** An apparatus according to claim 1 wherein the bone attachment means is non-metallic.

**33.** An apparatus according to claim 1 wherein the bone attachment means is a bone bolt that includes a proximal threaded section for receiving a nut.

**34.** An apparatus according to claim 1 wherein the bone attachment means is ceramic.

**35.** An apparatus according to claim 1 wherein the bone attachment means is composite.

**36.** A system according to claims 15 or 26 wherein the bone attachment means is metallic.

**37.** A system according to claims 15 or 26 wherein the bone attachment means is non-metallic.

**38.** A system according to claims 15 or 26 wherein the bone attachment means is a bone bolt that includes a proximal threaded section for receiving a nut.

**39.** A system according to claims 15 or 26 wherein the bone attachment means is ceramic.

**40.** A system according to claims 15 or 26 wherein the bone attachment means is composite.

**41.** A method of spinal fixation comprising the steps of:
a) providing a composite plate that includes non-metallic, fiber reinforced plate member;
b) affixing the plate member to a patient's spine using multiple, spaced apart fasteners that are surgically embedded in the patient's spine.

**42.** A load transfer washer adapted to interface with a plate member and a bone attachment means said washer being provided with an underside portion with projections extending therefrom adapted to correspond with openings in the limbs of said plate member.

# FIG.1.

# FIG.2.

# FIG.3.

# FIG.4.

# FIG.5.

# FIG. 6.

# FIG. 7.

# FIG. 8.

# FIG.9.

# FIG.10.

# FIG.11.

# FIG.12.

## FIG.13.

## FIG.16.

## FIG.14.

## FIG.17.

## FIG.15.

EP 0 637 437 A1

## PARTIAL EUROPEAN SEARCH REPORT

European Patent Office

which under Rule 45 of the European Patent Convention shall be considered, for the purposes of subsequent proceedings, as the European search report

Application Number

EP 94 30 3110

### DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X<br>Y | EP-A-0 506 420 (SMITH & NEPHEW RICHARDS)<br>* figures * | 42<br>1-3,<br>5-17,<br>19-28,<br>30-40 | A61B17/56 |
| Y | DE-A-29 33 659 (SULZER)<br><br><br>* page 2, line 3 *<br>* page 3, line 19 - line 26 * | 1-3,<br>5-17,<br>19-28,<br>30-40 | |
| A | EP-A-0 441 084 (J.-L.VIGNAUD AND P.HENRY)<br>* column 1, line 33 - line 36 *<br>* column 2, line 4 - line 6 *<br>* figure 3 * | 13-25,42 | |
| A | WO-A-90 13265 (AESCULAP)<br><br><br>* abstract; figures 1-3 *<br>---<br>-/-- | 2-5,<br>16-19,<br>27-30 | TECHNICAL FIELDS SEARCHED (Int.Cl.5)<br><br>A61B |

### INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:


see sheet C

| Place of search | Date of completion of the search | Examiner |
|---|---|---|
| THE HAGUE | 27 July 1994 | Nice, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

19

**European Patent Office**

## PARTIAL EUROPEAN SEARCH REPORT

**Application Number**

EP 94 30 3110

| | DOCUMENTS CONSIDERED TO BE RELEVANT | | CLASSIFICATION OF THE APPLICATION (Int.Cl.5) |
|---|---|---|---|
| Category | Citation of document with indication, where appropriate, of relevant passages | Relevant to claim | |
| A | EP-A-0 149 540 (ED.GEISTLICH SÖHNE)<br>* page 3, line 1 - line 4 *<br>* page 6, line 35 - line 37 *<br>--- | 4,18,29 | |
| A | DE-A-29 20 223 (M.A.N.)<br>* page 6, line 22 - page 7, line 5 *<br>* page 9, line 7 - line 15 *<br>----- | 3,17,28 | |

TECHNICAL FIELDS SEARCHED (Int.Cl.5)

EPO FORM 1503 03.82 (P04C10)

EP 0 637 437 A1

EP 94 30 3110

- C -

INCOMPLETE SEARCH

Claims searched completely : 1-40,42
Claims not searched        : 41

Reason  : Method for treatment of the human or animal
          body by surgery.

21